**Europäisches Patentamt**

**European Patent Office**

**Office européen des brevets**

(19)

(11) Veröffentlichungsnummer : **0 065 750**
**B1**

(12) # EUROPÄISCHE PATENTSCHRIFT

(45) Veröffentlichungstag der Patentschrift :
30.10.85

(51) Int. Cl.⁴ : **A 61 B  3/10**

(21) Anmeldenummer : **82104376.7**

(22) Anmeldetag : **19.05.82**

(54) **Tragbares ophthalmologisches Gerät zur wahlweisen Untersuchung der vorderen oder der hinteren Augenabschnitte.**

(30) Priorität : **27.05.81 DEU 8115734**

(43) Veröffentlichungstag der Anmeldung :
**01.12.82 Patentblatt 82/48**

(45) Bekanntmachung des Hinweises auf die Patenterteilung : **30.10.85 Patentblatt 85/44**

(84) Benannte Vertragsstaaten :
**CH FR GB IT LI**

(56) Entgegenhaltungen :
**EP-A- 0 029 203**
**EP-A- 0 041 107**
**FR-A- 1 021 272**
**FR-A- 2 163 302**
**GB-A- 2 053 502**
**US-A- 1 887 115**
**US-A- 3 904 280**
**US-A- 3 945 712**

(73) Patentinhaber : **Firma Carl Zeiss**

**D-7920 Heidenheim (Brenz) (DE)**

(72) Erfinder : **Blaha, Erich**
**Staufenstrasse 15**
**D-7081 Essingen (DE)**

## Beschreibung

Die vorliegende Erfindung betrifft ein tragbares ophtalmologisches Gerät zur wahlweisen Untersuchung der vorderen oder der hinteren Augenabschnitte.

Es ist bekannt zur Fundusuntersuchung im umgekehrten Bild, d. h. also zur Untersuchung des hinteren Augenabschnitts eine tragbare Ophtalmoskopier leuchte zu verwenden. Diese besteht aus einem stabförmigen Gehäuse, das eine Lichtquelle, einen Kondensor, eine Irisblende, eine Scheibe zum wahlweisen Einbringen eines Farbfilters, ein Objektiv sowie an der Lichtaustrittsseite ein Umlenkprisma enthält. Der Arzt hält zur Untersuchung diese Ophthalmoskopierleuchte in der einen Hand und hält mit der anderen Hand eine Ophthalmoskopierlupe vor das Auge des Patienten.

Um die Beobachtung zu erleichtern, ist auf die bekannte Ophthalmoskopierleuchte ein sogenannter Stereobetrachter aufschiebbar. Der Arzt kann dann nach Aufschieben dieses Zusatzteiles den hinteren Augenabschnitt stereoskopisch betrachten.

Eine Beobachtung der vorderen Augenabschnitte ist mit dieser bekannten Ophthalmoskopierleuchte nicht möglich. Diese Augenabschnitte werden üblicherweise mittels einer stationär angeordneten Spaltlampe untersucht.

Bekannt ist auch eine Handspaltlampe mit einem Binokularmikroskop. Bei diesem bekannten tragbaren Gerät ist seitlich vom Binokularmikroskop in einem festen Winkel zu dessen optischer Achse eine Spaltlampenbeleuchtung angeordnet. Diese erzeugt in dem zu beobachtenden vorderen Augenabschnitt ein Spaltbild, das mittels des Binokularmikroskops stereoskopisch beobachtet wird. Das Gerät ist mit einer Wangenstütze versehen, die am unteren, knöchernen Rand der Augenhöhle des Patienten aufgesetzt wird. Zum Zwecke der Fokussierung ist diese Auflage in Achsrichtung bewegbar. Bei der Untersuchung des Patienten wird zum Absuchen der Oberfläche des Auges das gesamte Gerät bewegt, wobei die Wangenstütze als Angelpunkt benützt wird.

Diese bekannte Handspaltlampe ist nur zur Untersuchung der vorderen Augenabschnitte geeignet.

Es ist nun die Aufgabe der vorliegenden Erfindung ein tragbares ophthalmologisches Gerät zur wahlweisen Untersuchung der vorderen und der hinteren Augenabschnitte zu schaffen, das preiswert ist und das mit wenigen Handgriffen für die verschiedenen Untersuchungen umrüstbar ist.

Diese Aufgabe wird, ausgehend von einer, in einem stabförmigen Gehäuse untergebrachten Ophthalmoskopierleuchte, die an ihrer Lichtaustrittsseite ein Umlenkprisma aufweist, dadurch gelöst, daß ein Stereomikroskop fest mit einer Kreisbogenführung verbunden ist, in der eine Hülse verschiebbar gelagert ist, daß diese Hülse auf das stabförmige Gehäuse aufschiebbar und mit diesem verbindbar ist und eine Zusatzlinse trägt, die nach dem Aufschieben der Hülse vor dem Umlenkprisma der Ophthalmoskopierleuchte angeordnet ist, und daß das Gehäuse dieser Leuchte zusätzlich mit einer Scheibe zum wahlweisen Einbringen einer Kreis- und mindestens einer Spaltblende in den Beleuchtungsstrahlengang versehen ist.

Wird bei dem Gerät nach der Erfindung die im stabförmigen Gehäuse untergebrachte Ophthalmoskopierleuchte alleine verwendet, so wird die Kreisblende in den Beleuchtungsstrahlengang eingeschwenkt und die indirekte Ophthalmoskopie, d. h. die Beobachtung der hinteren Augenabschnitte mit Hilfe einer Ophthalmoskopierlupe durchgeführt.

Zur Untersuchung der vorderen Augenabschnitte wird die in der Kreisbogenführung des Stereomikroskops geführte Hülse auf das Gehäuse der Ophthalmoskopierleuchte aufgeschoben. Dabei wird automatisch vor dem Umlenkprisma dieser Leuchte eine Zusatzlinse angeordnet. In den Beleuchtungsstrahlengang der Ophthalmoskopierleuchte wird zugleich eine Spaltblende eingeschwenkt. Diese wird jetzt mit Hilfe der Zusatzlinse in die vorderen Augenabschnitte abgebildet.

Zur Untersuchung hält der Arzt das gesamte kombinierte Gerät in der Hand und setzt die Auflage des Abstandhalters auf die Stirn des Patienten auf. Durch leichtgängiges Verändern der Länge des Abstandshalters wird auf den jeweils zu untersuchenden Augenabschnitt fokussiert und die dabei ermittelte Länge des Abstandshalters wird fixiert.

Die Ophthalmoskopierleuchte wirkt bei der beschriebenen Anwendung als Spaltlampenbeleuchtung. Das von ihr erzeugte Spaltbild kann durch Verschieben des Leuchtengehäuses in der Kreisbogenführung des Stereomikroskops in einem bestimmten Winkelbereich um das Auge des Patienten verschwenkt werden.

Um zu vermeiden, daß bei einer, bei Untersuchung am liegenden Patienten ohne weiteres möglichen Schrägstellung des gesamten Gerätes eine unbeabsichtigte Verschwenkung des Spaltbildes auftritt ist die Kreisbogenführung mit mehreren Raststellungen für die Hülse versehen.

Die Erfindung wird im folgenden anhand der Figuren 1 bis 5 der beigefügten Zeichnungen näher erläutert. Im einzelnen zeigen :

Figur 1 eine schematische Darstellung der optischen Elemente des Gerätes nach der Erfindung ;

Figur 2 eine Seitenansicht einer Ophthalmoskopierleuchte ;

Figur 3 eine Draufsicht auf ein nach einem Ausführungsbeispiel der Erfindung ausgebildetes Stereomikroskop ;

Figur 4 die Geräte der Fig. 2 und 3 in kombiniertem Zustand ;

Figur 5 eine Draufsicht auf die Ophthalmoskopierleuchte in der Kombination nach Fig. 4.

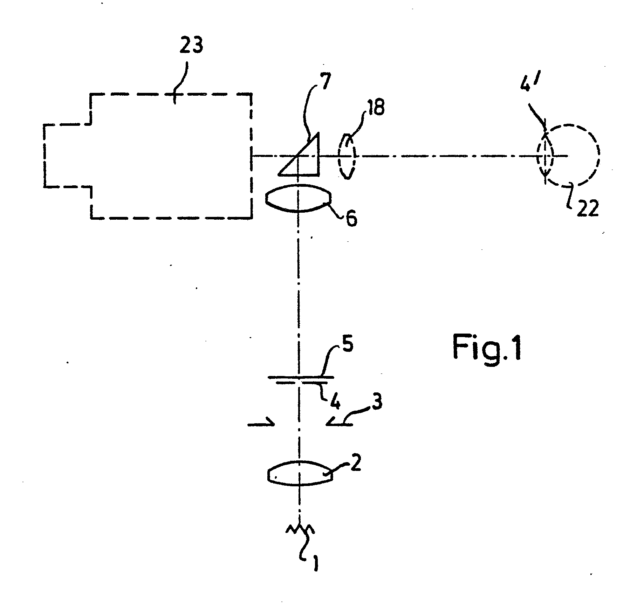

In Fig. 1 ist mit 1 eine Lichtquelle und mit 2 ein Kondensor bezeichnet. Im Strahlengang ist eine verstellbare Irisblende 3, eine einschwenkbare Blende 4 und ein einschwenkbares Filter 5 angeordnet. Das durch diese Elemente tretende Licht wird mittels eines fest angeordneten Objektivs 6 und eines Umlenkprismas 7 so in das zu untersuchende Auge abgebildet, daß dessen Netzhaut gleichmäßig beleuchtet ist. Mittels einer, vorzugsweise asphärisch ausgebildeten Ophthalmoskopierlupe betrachtet der Arzt dann nach dem bekannten Prinzip der indirekten Ophthalmoskopie das Patientenauge. Die Blende 4 ist hier als Kreisblende ausgebildet, welche die durch die Irisblende 3 tretenden Strahlen nicht abschattet.

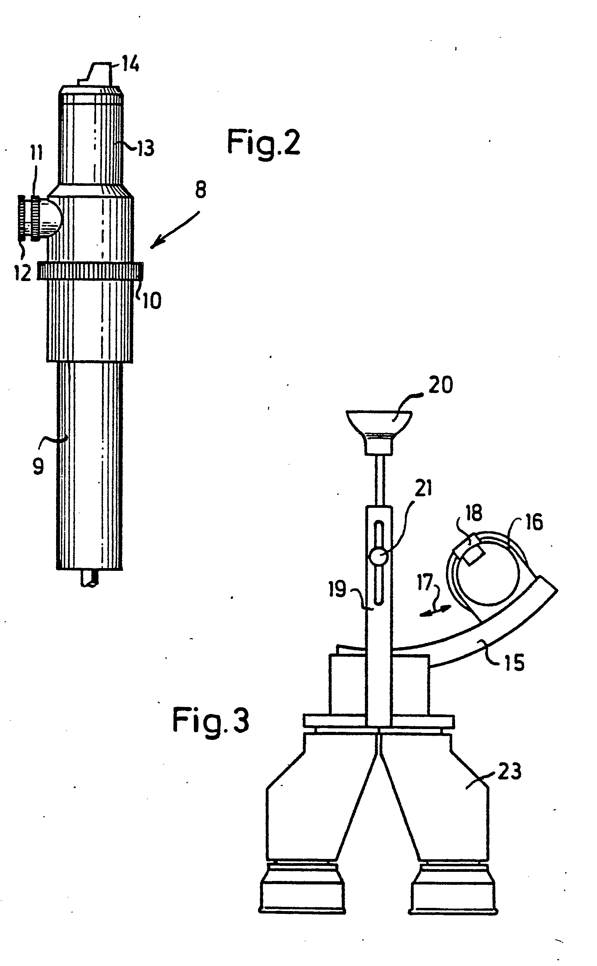

Fig. 2 zeigt das stabförmige Gehäuse 8 in dem die im Zusammenhang mit Fig. 1 beschriebenen Elemente angeordnet sind. Im unteren Gehäuseteil 9 ist beispielsweise ein Netzgerät oder ein Akkublock zur Stromversorgung der Lampe 1 angeordnet. Dieser Gehäuseteil wird vom Arzt in der Hand gehalten. Mit 10 ist ein Ring zum Verstellen der Irisblende 3 bezeichnet. Eine Scheibe 12 dient zum Einschwenken einer vorgewählten Blende. Diese kann als Kreis- oder als Spaltblende ausgebildet sein. Die Scheibe 11 dient zum Einschwenken eines vorgewählten Farbfilters, z. B. eines Grün- oder Blaufilters. Die Farbfilter 5 und die Blenden 4 sind zweckmäßig in koaxial angeordneten, hier nicht dargestellten, Rekosshülsen angeordnet.

Der obere Gehäuseteil 13 ist zylindrisch ausgebildet und trägt in einer Fassung 14 das Umlenkprisma 7.

In Fig. 3 ist mit 23 ein Stereomikroskop bezeichnet, das zwei auf den Augenabstand des Beobachters einstellbare Okulartuben und ein Mikroskopobjektiv enthält. Mit dem Gehäuse dieses Stereomikroskops 23 ist eine Kreisbogenführung 14 fest verbunden, welche wahlweise für Links- oder Rechtsbenutzung angeordnet werden kann. In dieser ist eine Hülse 16 in Richtung des Pfeils 17 verschiebbar gelagert. Die Hülse 16 trägt eine Zusatzlinse 18.

Mit dem Stereomikroskop 23 ist weiterhin ein Abstandshalter 19 fest verbunden, dessen Länge federnd einstellbar ist. Zur Fixierung einer vorbestimmten Länge des Abstandshalters 19 dient eine Klemmschraube 21. Am vorderen Ende des Halters 19 ist eine Auflage 20 aus weichem Material vorgesehen.

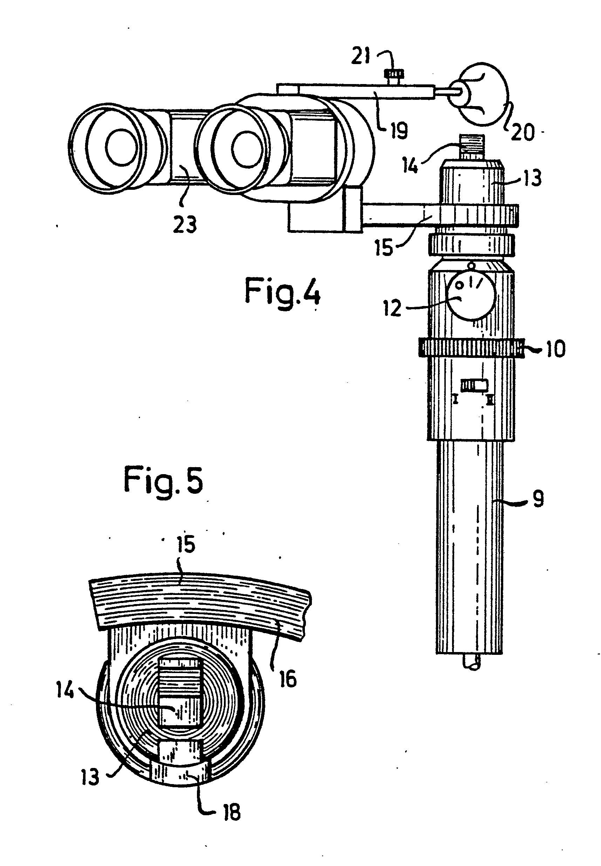

Soll das ophthalmologische Gerät nach der Erfindung als Handspaltlampe verwendet werden, so wird die mit dem Stereomikroskop 23 verbundene Hülse 16 auf den oberen Teil 13 des stabförmigen Gehäuses 8 aufgeschoben und z. B. mit Hilfe eines Bayonetts mit diesem fest verbunden. Die so entstehende Handspaltlampe zeigt Fig. 4 ; der optische Aufbau ist gestrichelt in Fig. 1 eingezeichnet.

Beim Aufschieben der Hülse 16 auf den Gehäuseteil 13 wird, wie Fig. 5 zeigt, die mit der Hülse 16 verbundene Zusatzlinse 18 automatisch vor dem Gehäuse 14 des Umlenkprismas 7 angeordnet.

Wird nun mittels der Scheibe 12 eine Spaltblende in den Beleuchtungsstrahlengang gebracht, wie dies in Fig. 4 gezeigt ist, so entsteht ein Bild 4' der Spaltblende 4 in dem zu untersuchenden vorderen Augenabschnitt des Patientenauges 22. Dieses Bild wird mittels des Stereomikroskops 23 beobachtet. Zur Untersuchung des Patienten wird die Auflage 20 auf die Stirn des Patienten aufgesetzt und durch Verändern der Länge des Abstandshalters 19 wird auf den zu beobachtenden Augenabschnitt fokussiert.

Die Kreisbogenführung 15 weist mehrere Raststellungen für die Hülse 16 auf, um deren unbeabsichtigtes Verstellen zu vermeiden.

Das Gerät nach der Erfindung kann auch als Binokularophthalmoskop ausgebildet werden. Dazu ist es notwendig die Zusatzlinse 18 ausschwenkbar anzuordnen. Zur Untersuchung wird dann beim kombinierten Gerät nach Fig. 4 mittels der Scheibe 12 eine Kreisblende gewählt, die Linse 18 ausgeschwenkt und die Ophthalmoskopierleuchte im Gehäuse 8 in der Kreisbogenführung 15 solange verschwenkt, bis das Umlenkprisma 14 zwischen den Ausblicköffnungen des Stereomikroskops 23 liegt.

**Patentansprüche**

1. Tragbares ophthalmologisches Gerät zur wahlweisen Untersuchung der vorderen oder der hinteren Augenabschnitte mit einer in einem stabförmigen Gehäuse untergebrachten Ophthalmoskopierleuchte die an ihrer Lichtaustrittseite ein Umlenkprisma aufweist, dadurch gekennzeichnet, daß ein Stereomikroskop (23) fest mit einer Kreisbogenführung (15) verbunden ist in der eine Hülse (16) verschiebbar gelagert ist, daß diese Hülse (16) auf das stabförmige Gehäuse (13) aufschiebbar und mit diesem verbindbar ist und eine Zusatzlinse (18) trägt, die nach dem Aufschieben der Hülse (16) vor dem Umlenkprisma (7) der Ophthalmoskopierleuchte (8) angeordnet ist, und daß das Gehäuse (8) dieser Leuchte zusätzlich mit einer Scheibe (12) zum wahlweisen Einbringen einer Kreis- und mindestens einer Spaltblende (4) in den Beleuchtungsstrahlengang versehen ist.

2. Tragbares ophthalmologisches Gerät nach Anspruch 1, dadurch gekennzeichnet, daß die Kreisbogenführung (15) mehrere Raststellungen für die Hülse (16) aufweist.

3. Tragbares ophthalmologisches Gerät nach Anspruch 1 und 2, dadurch gekennzeichnet, daß mit dem Stereomikroskop (23) ein mit einer Stirnauflage (20) versehener, federnd in der Länge verstellbarer Abstandshalter (19) verbunden ist.

4. Tragbares ophthalmologisches Gerät nach Anspruch 3, dadurch gekennzeichnet, daß der Abstandshalter (19) eine Schraube (21) zur Fixierung des Abstandes aufweist.

3

5. Tragbares ophthalmologisches Gerät nach Anspruch 1, dadurch gekennzeichnet, daß die Zusatzlinse (18) ausschwenkbar angeordnet ist.

**Claims**

1. A portable ophthalmological instrument for selective examination of anterior or posterior regions of the eye, comprising an ophthalmoscopy arranged within a tubular housing and having a deflection prism at its light-exit side, characterized in that a stereomicroscope (23) is firmly connected with an arcuate guide (15) within which a sleeve (16) is displaceably supported, that said sleeve is slidably over said tubular housing (13) and connectable to this housing, that said sleeve (16) carries a supplementary lens (18) which upon sliding of the sleeve (16) over the tubular housing (13) is positioned in front of the deflection prism (7) of the ophthalmoscopy light source (8) and that the housing (8) of said light source is further provided with a knob (12) for optionally bringing a circular diaphragm and at least one slit diaphragm (4) into the illumination-ray path.

2. A portable ophthalmological instrument according to claim 1, characterized in that the arcuate guide (15) has a plurality of detent positions for the sleeve (16).

3. A portable ophthalmological instrument according to claims 1 and 2, characterized in that a spacer (19) is connected with the stereomicroscope (23), which is equiped with a forehead resting (20) and which is spring-loaded adjustable in length.

4. A portable ophthalmological instrument according to claim 3, characterized in that the spacer (19) includes a screw (21) for fixing an adjusted spacer length.

5. A portable ophthalmological instrument according to claim 1, characterized in that the supplementary lens (18) is arranged in such a manner that it can be swung out.

**Revendications**

1. Appareil ophtalmologique portatif pour l'examen, au choix, du segment antérieur ou du segment postérieur de l'œil, comprenant une lampe d'ophtalmoscopie dans un corps en forme de bâton et présentant côté sortie de la lumière un prisme déviateur, caractérisé en ce qu'un stéréomicroscope (23) est relié fixe à un guide en arc de cercle (15) dans lequel une douille (16) est montée coulissante, que cette douille (16) peut être emboîtée et fixée sur le corps en forme de bâton (13) de la lampe et porte une lentille additionnelle (18) qui est située devant le prisme déviateur (7) de la lampe d'ophtalmoscopie (8) après emboîtement de la douille (16), et que le corps (8) de cette lampe est équipée en outre d'un disque (12) pour l'introduction sélective d'un diaphragme circulaire et d'au moins un diaphragme à iris (4) dans le faisceau d'éclairement.

2. Appareil ophtalmologique portatif selon la revendication 1, caractérisé en ce que le guide en arc de cercle (15) présente plusieurs positions d'arrêt pour la douille (16).

3. Appareil ophtalmologique portatif selon les revendications 1 et 2, caractérisé en ce qu'une entretoise (19) dont la longueur est élastiquement réglable et qui est pourvue d'un appui (20) contre le front, est reliée au stéréomicroscope (23).

4. Appareil ophtalmologique selon la revendication 3, caractérisé en ce que l'entretoise (19) présente une vis (21) pour la fixation de la distance.

5. Appareil ophtalmologique portatif selon la revendication 1, caractérisé en ce que la lentille additionnelle (18) est disposée de manière à pouvoir être écartée par pivotement.

Fig.1

Fig.2

Fig.3

Fig.4

Fig.5